(19) Europäisches Patentamt
European Patent Office
Office européen des brevets

(11) EP 2 898 905 B1

# (12) EUROPEAN PATENT SPECIFICATION

(45) Date of publication and mention of the grant of the patent:
**20.12.2017 Bulletin 2017/51**

(21) Application number: **13838791.5**

(22) Date of filing: **19.09.2013**

(51) Int Cl.:
***A61L 29/04*** *(2006.01)* ***A61L 33/06*** *(2006.01)*

(86) International application number:
**PCT/JP2013/075250**

(87) International publication number:
**WO 2014/046158 (27.03.2014 Gazette 2014/13)**

## (54) PELLET-SHAPED COMPOSITION FOR MEDICAL USE, AND MOLDED ARTICLE

PELLETFÖRMIGE ZUSAMMENSETZUNG ZUR MEDIZINISCHEN VERWENDUNG UND FORMKÖRPER

COMPOSITION EN FORME DE PASTILLE POUR UNE UTILISATION MÉDICALE, ET ARTICLE MOULÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.09.2012 JP 2012206783**

(43) Date of publication of application:
**29.07.2015 Bulletin 2015/31**

(73) Proprietor: **Toyobo Co., Ltd.**
**Osaka-shi**
**Osaka 530-8230 (JP)**

(72) Inventors:
- **KAWAKATSU, Yuta**
**Osaka-shi**
**Osaka 5308230 (JP)**
- **KARATO, Yoshiaki**
**Yokkaichi-shi**
**Mie 510-0106 (JP)**
- **OHASHI, Tomoya**
**Otsu-shi**
**Shiga 5200292 (JP)**
- **KASHIWABARA, Susumu**
**Osaka-shi**
**Osaka 5308230 (JP)**

(74) Representative: **Vossius & Partner**
**Patentanwälte Rechtsanwälte mbB**
**Siebertstrasse 3**
**81675 München (DE)**

(56) References cited:
**WO-A1-2008/068868**
**WO-A1-2011/083815**
**JP-A- S4 722 451**
**JP-A- S5 887 143**
**JP-A- S6 116 952**
**JP-A- S6 116 952**
**JP-A- H06 212 078**
**JP-A- H11 164 882**
**JP-A- S61 145 243**
**JP-A- 2003 165 881**
**JP-A- 2005 089 605**
**JP-A- 2007 146 133**
**JP-A- 2008 264 266**
**JP-A- 2008 289 864**
**JP-A- 2009 261 437**
**JP-A- 2009 261 437**
**US-A- 4 478 961**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 898 905 B1

## Description

### Technical Field of the Invention

The present invention relates to a pellet-shaped composition for medical use which can be suitably used for main tube, etc. constituting a blood circuit for artificial dialysis or heart-lung apparatus. More particularly, it relates to a pellet-shaped composition for medical use which contains a polyvinyl chloride resin, a plasticizer and a copolymer of hydrophobic (meth)acrylate with hydrophilic (meth)acrylate and which can manufacture a medical material exhibiting excellent antithrombotic property and transparency.

### Background Art

In recent years, investigations for medical materials utilizing various kinds of synthetic polymer materials have been carried out. Such medical materials have been utilized for blood filters, membranes for artificial kidney, membranes for plasma separation, catheters, membranes for artificial lung, artificial blood vessels, membrane for prevention of adhesion, artificial skins, tubes for medical use, etc. A tube for medical use has been used for very many medical devices such as an infusion set for administration of infusion fluid from an infusion bag to human, a blood transfusion set used in the same manner upon blood transfusion, a blood bag used for collection of blood from human upon blood donation or the like, and a circuit which is used for blood dialysis or upon the use of heart-lung apparatus, etc.

Up to now, polyurethane resin, silicone resin and vinyl chloride resin have been commonly used as materials for molding a tube for medical use. Among them, a tube for medical use comprising vinyl chloride resin has such characteristics that good molding ability, less expensiveness for materials and for manufacturing cost, appropriate soft property as a tube, good processing ability for assembling into medical devices, etc.

In addition, a tube for medical use is required to have biocompatibility (antithrombotic property) since synthetic polymer materials which are foreign bodies to living organisms are used by contacting with *in vivo* tissues or with blood. When a tube for medical use is used as a material for contacting with blood, the following three elements are important items as biocompatibility: (a) suppression of blood coagulation system; (b) suppression of adhesion/activation of platelets; and (c) suppression of activation of complement system. Particularly when it is used in such a use where contacting time with blood is relatively short such as artificial kidney or plasma separation membrane, an anticoagulant such as heparin or sodium citrate is commonly used at the same time and, accordingly, suppression of activation of platelets (b) and that of complement system (c) are important aims.

The present applicant has already carried out various investigations for a purpose of imparting the biocompatibility (antithrombotic property) to medical materials such as a tube for medical use. Up to now, the present applicant has been already filed patent applications relating an art wherein heparin or heparin derivative and a (meth)acrylate copolymer containing hydrophilic and hydrophobic group are coated (fixed) on the surface of a tube (for example, Patent Documents 1 to 3). However, when the material as mentioned above is dissolved in organic solvent and coated on the surface of a tube for medical use by means of an after-coating, there have been problems of causing appearance defect such as that a plasticizer is eluted out, that turbidity is generated on the surface of a tube due to the property of the material itself or that oil defect is resulted.

On the other hand, an art wherein a hydrophobic (meth) acrylate polymer is added as a plasticizer for a polyvinyl chloride resin has been known already (for example, Patent Document 4). According to the document, a (meth)acrylate copolymer is used for a purpose of improvement of shock resistance and weather resistance of the polyvinyl chloride resin. However, since the monomer used therefor is an alkyl (meth)acrylate only which is hydrophobic, the resulting antithrombotic property is insufficient. In Patent Document 5 a (meth)acrylate polymer is added to improve the extrusion properties.

### Prior Art Documents

#### Patent Documents

Patent Document 1: Japanese Patent (JP-B) No. 3228409
Patent Document 2: Japanese Patent (JP-B) No. 4162028
Patent Document 3: Japanese Patent (JP-B) No. 4793700
Patent Document 4: Japanese Patent Application Laid-Open (JP-A) No. 2005-89605
Patent Document 5: JP-S6116952 A

Disclosure of the Invention

Problem that the Invention is to Solve

A problem that the present invention is to solve is to provide a pellet-shaped composition for medical use containing a polyvinyl chloride resin, a plasticizer and a (meth)acrylate copolymer which contains a hydrophilic (meth) acrylate unit and a hydrophobic (meth) acrylate unit and achieving both transparency and antithrombotic property when molded into a tube for medical use, etc.

Means for Solving the Problem

In order to solve the above-mentioned conventional problems, the present inventors have conducted intensive investigations and, as a result, they have at last achieved the present invention. Thus, the present invention has the following constitutions.

(1) A pellet-shaped composition for medical use which comprises 1 to 120 part (s) by weight of a plasticizer and 0.01 to 5 part (s) by weight of a (meth) acrylate copolymer containing a hydrophobic (meth)acrylate unit and a hydrophilic (meth)acrylate unit to 100 parts by weight of a polyvinyl chloride resin, wherein number-average molecular weight of the (meth)acrylate copolymer is 7,000 to 50,000, and the hydrophobic (meth)acrylate unit and the hydrophilic (meth)acrylate unit are copolymerized in a molar ratio of 55 - 80 to 20 - 45.

(2) The pellet-shaped composition for medical use according to (1), wherein the hydrophobic (meth)acrylate unit contains an alkyl (meth)acrylate unit represented by the following formula 1 and/or a silicone (meth)acrylate unit represented by the following formula 2:

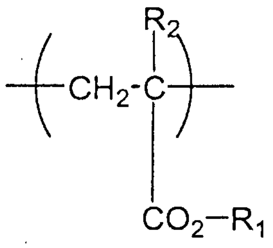

(1)

wherein, $R_1$ is an alkyl group or an aralkyl group having 6 to 14 carbon atoms and $R_2$ represents hydrogen atom or methyl group;

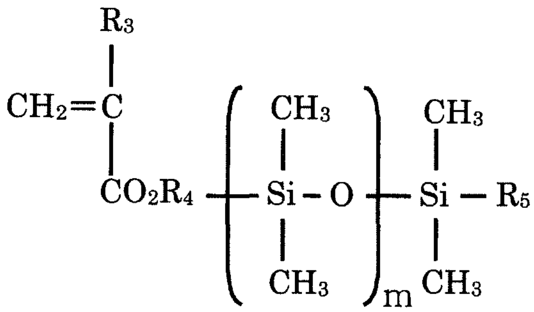

(2)

wherein, $R_3$ represents hydrogen atom or methyl group, $R_4$ represents an alkylene group having 1 to 6 carbon atom(s), $R_5$ represents an alkyl group having 1 to 6 carbon atom(s) and m is within a range of 1 to 100.

(3) The pellet-shaped composition for medical use according to (1) or (2), wherein the hydrophilic (meth)acrylate unit contains a methoxy polyethylene glycol (meth) acrylate unit represented by the formula 3:

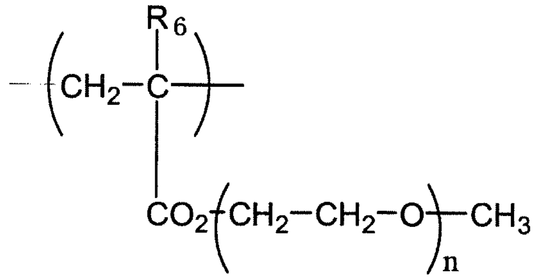

(3)

wherein, $R_6$ represents hydrogen atom or methyl group and m is an integer of 2 to 4.

(4) The pellet-shaped composition for medical use according to any of (1) to (3), wherein number-average polymerization degree of the polyvinyl chloride resin is 700 to 3,000.

(5) The pellet-shaped composition for medical use according to any of (1) to (4), wherein the plasticizer is one or more member (s) selected from di-2-ethylhexyl phthalate (DOP), tri-2-ethylhexyl trimellitate (TOTM) and diisononyl cyclohexane-1,2-dicarboxylate (DINCH).

(6) A molded product prepared by melt molding the pellet-shaped composition for medical use mentioned in any of (1) to (5).

(7) A tube for medical use prepared by melt molding the pellet-shaped composition for medical use mentioned in any of (1) to (5).

Advantages of the Invention

The pellet-shaped composition for medical use according to the present invention can provide a medical material having excellent antithrombotic property and high hydrophilicity when the composition is molded into the medical material. In addition, since no turbidity is resulted in the molded product, a molded product having good appearance can be produced and good visibility can be ensured in the field of operation.

Brief Description of the Drawings

Fig. 1 is a schematic drawing which shows the cause for turbidity of a pellet-shape composition for medical use.

Fig. 2 is a schematic drawing which shows the range achieving good transparency of a pellet-shape composition for medical use.

Fig. 3 is a schematic drawing which shows the range achieving good antithrombotic property of a pellet-shape composition for medical use.

Fig. 4 is a schematic drawing of an extrusion molding machine for the manufacture of a tube for medical use using a pellet-shaped composition for medical use.

Fig. 5 is a schematic drawing which shows the process for measuring the water drop width.

Best Mode for Carrying Out the Invention

As hereunder, modes for carrying out the pellet-shaped composition for medical use according to the present invention and also a molded product thereof comprising the same will be more particularly illustrated.

An object of the present invention is to impart both antithrombotic property and transparency to a pellet-shaped composition for medical use comprising a polyvinyl chloride resin and a plasticizer such as DOP or TOTM. The present inventors have achieved the present invention by means of a combination of the following four steps, i.e. (I) a step for imparting an antithrombotic property to a pellet-shaped composition for medical use, (II) a step for analyzing the cause for making the pellet-shaped composition for medical use turbid, (III) a step for preparing a (meth) acrylate copolymer which does not make the pellet-shaped composition for medical use turbid and (IV) a step for achieving both antithrombotic property and transparency in the pellet-shaped composition for medical use. As hereunder, each of those steps will be illustrated in detail. Since there is a difference in the compositions of the (meth)acrylate copolymer, a (meth)acrylate copolymer before changing the composition will be referred to as a (meth) acrylate copolymer (A) while that after changing the composition will be referred to as a (meth)acrylate copolymer (B).

Firstly, "(I) a step for imparting an antithrombotic property to a pellet-shaped composition for medical use" will be illustrated. The present inventors prepared a pellet-shaped antithrombotic composition for medical use by adding a (meth) acrylate copolymer (A) (which is an antithrombotic material) to polyvinyl chloride and a plasticizer, as a result of

application of the fact that poly(alkyl(meth)acrylate) has been utilized as a plasticizer for a polyvinyl chloride resin already. However, the resulting pellet-shaped composition for medical use has been found to exhibit no commercial value since it becomes turbid due to failure of uniform mixing. In addition, it has been also found that the antithrombotic property is lost when the amount of a (meth)acrylate copolymer (A) added thereto is reduced to such an extent that no turbidity is resulted.

"(II) A step for analyzing the cause for making the pellet-shaped composition for medical use turbid" will be illustrated. As shown in Fig. 1, the present inventors have presumed that a hydrophobic (meth) acrylate is apt to be mixed with a polyvinyl chloride resin and a hydrophobic component such as DOP while, upon mixing with a hydrophilic (meth)acrylate, it forms a micelle structure of a core/shell type having inner area of a hydrophilic core and outer area of a hydrophobic shell in the resulting pellet-shaped composition for medical use, which is the cause of turbidity. For eliminating the turbidity, it is necessary to make the micelle small to such an extent that it is no longer visible by naked eye.

"(III) A step for preparing a (meth)acrylate copolymer which does not make the pellet-shaped composition for medical use turbid" will be illustrated. The present inventors have prepared a transparent pellet-shaped composition for medical use by addition of a (meth)acrylate copolymer (B) wherein all of items of chain length of polyethylene glycol, composition ratio of a hydrophilic (meth)acrylate and number-average molecular weight of (meth)acrylate copolymer (A) are optimized within such an extent that a pellet-shaped composition for medical use can still maintain its antithrombotic property. To be more specific, the chain length of polyethylene glycol and the composition ratio of hydrophilic (meth) acrylate are lowered as a result of judgment that lowering of the hydrophilicity is effective. In addition, number-average molecular weight of a (meth)acrylate copolymer (A) is lowered for a purpose of lowering the polyethylene glycol numbers in a micelle. When hydrophilicity became high even only a little, a pellet-shaped composition for medical use became turbid. Accordingly, it has been found that a pellet-shaped composition for medical use results in turbidity even if any of the elements becomes out of the suitable range. The reason why a transparent pellet-shaped composition for medical use has been prepared is presumed to be due to such a cause that the micelle became so small that it is no longer visible by naked eye, as mentioned above. However, it has been also found that, even in the case of the (meth) acrylate copolymer (B) prepared as such, a pellet-shaped composition for medical use becomes turbid if it is added in an amount of more than the predetermined extent.

"(IV) A step for achieving both antithrombotic property and transparency in the pellet-shaped composition for medical use" will be illustrated. The present inventors have added a (meth)acrylate copolymer (B) in an amount which can achieve both of the transparency and the antithrombotic property to a polyvinyl chloride resin and a plasticizer whereby they have achieved the present invention.

In the present invention, number-average molecular weight of a (meth) acrylate copolymer is preferred to be 50,000 or less. Within such a range, a micelle comprising the (meth)acrylate copolymer becomes sufficiently small whereby a transparent pellet-shaped composition for medical use can be prepared. When it is less than 7,000, the (meth) acrylate copolymer is apt to be eluted into blood and a reduction of the antithrombotic property becomes significant whereby that is not preferred. Thus, it is preferred to be 7,000 or more, more preferred to be 8, 000 or more, and further preferred to be 9, 000 or more. Examples of a method for measuring the number-average molecular weight are a quantitative method for terminal group, an osmotic pressure method, a vapor pressure osmometry, a vapor pressure depression method, a freezing-point depression method, a boiling-point elevation method and a gel permeation chromatography (GPC) and, in the present invention, a gel permeation chromatography (GPC) is adopted due to its easy operation.

As to an alkyl (meth)acrylate represented by the following formula 1 which is the hydrophobic (meth) acrylate of the present invention, it is preferred to use such a one having carbon atom number of $R_1$ of 6 to 14, and more preferred to use such a one having carbon atom number of $R_1$ of 8 to 12. Specific examples of the alkyl (meth)acrylate as such include n-hexyl (meth)acrylate, cyclohexyl (meth)acrylate, heptyl (meth)acrylate, octyl (meth)acrylate, 2-ethylhexyl (meth)acrylate, nonyl (meth)acrylate, decyl (meth)acrylate and lauryl (meth) acrylate. When carbon atom number thereof is too small, hydrophobicity becomes insufficient and there is a risk that the alkyl (meth)acrylate cannot be uniformly dissolved in a polyvinyl chloride resin and a plasticizer, which results in turbidity while, when the carbon atom number is too large, hydrophilicity lowers and there is a risk that antithrombotic property of the pellet-shaped composition for medical use lowers. Thus, 2-ethylhexyl (meth)acrylate and lauryl (meth)acrylate are particularly preferred.

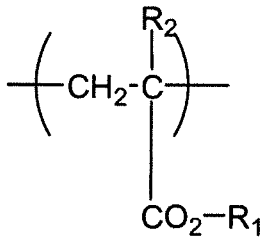

(1)

wherein, $R_1$ is an alkyl group or an aralkyl group having 6 to 14 carbon atoms and $R_2$ represents hydrogen atom or methyl group.

As to a silicone (meth) acrylate represented by the following formula 2 which is the hydrophobic (meth) acrylate of the present invention, it is preferred to use a silicone (meth)acrylate having 1 to 100 dimethylsiloxane repeating unit(s). When number of repeating unit(s) is too small, hydrophobicity becomes insufficient and there is a risk that the silicone (meth)acrylate cannot be uniformly dissolved in a polyvinyl chloride resin and a plasticizer, which results in turbidity while, when the number of repeating unit(s) is too large, hydrophilicity lowers and there is a risk that antithrombotic property of the pellet-shaped composition for medical use lowers. Accordingly, number of the dimethyl siloxane repeating unit is more preferred to be 5 to 100, further preferred to be 10 to 50, and further more preferred to be 10 to 30. When the silicone (meth)acrylate is used as a hydrophobic monomer, there is also achieved a secondary effect that a water repelling property is enhanced and accordingly that hydrolysis of the (meth)acrylate copolymer can be suppressed.

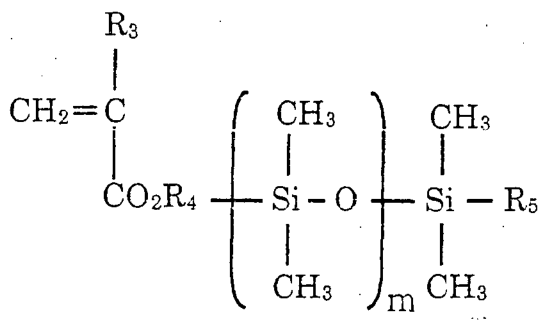

(2)

wherein, $R_3$ represents hydrogen atom or methyl group, $R_4$ represents an alkylene group having 1 to 6 carbon atom(s), $R_5$ represents an alkyle group having 1 to 6 carbon atom(s) and m is within a range of 1 to 100.

As to a methoxy polyethylene glycol (meth) acrylate represented by the following formula 3 which is the hydrophilic (meth) acrylate of the present invention, it is preferred to use such a one which has 2 to 4 ethylene oxide units. Specific examples thereof include methoxy diethylene glycol (meth)acrylate, methoxy triethylene glycol (meth)acrylate and methoxy tetraethylene glycol (meth)acrylate. When the repeating unit is big and hydrophilicity becomes too high, the pellet-shaped composition for medical use becomes turbid while, when it is too small, hydrophobicity becomes too high whereby there is a possibility that no antithrombotic property is achieved. Accordingly, methoxy triethylene glycol (meth) acrylate having 3 ethylene oxide units is more preferred.

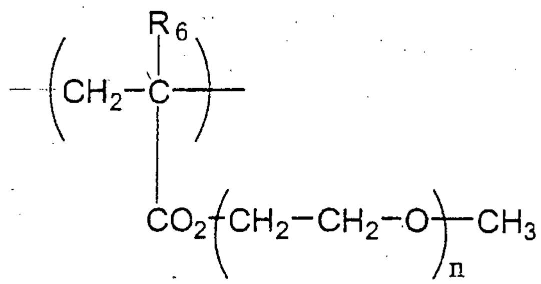

(3)

wherein, $R_6$ represents hydrogen atom or methyl group and m is an integer of 2 to 4.

In the present invention, the hydrophobic (meth)acrylate unit and the hydrophilic (meth)acrylate are copolymerized in a molar ratio of 55-80 to 20-45. When the molar ratio of methoxy polyethylene glycol (meth)acrylate which is a hydrophilic monomer is within this range, both of the antithrombotic property and the transparency of a pellet-shaped

composition for medical use can be achieved.

Adding amount of the (meth)acrylate copolymer of the present invention is preferred to be 0.01 to 5 part (s) by weight to 100 parts by weight of a polyvinyl chloride resin. The term reading "part(s) by weight" used herein means a compounding amount when the weight of a polyvinyl chloride resin is 100. When adding amount of the (meth) acrylate copolymer is within such a range, both of the antithrombotic property and the transparency of a pellet-shaped composition for medical use can be achieved whereby that is preferred. When it is too small, there is a risk that no antithrombotic property is achieved whereby that is not preferred while, when it is too much, cost becomes high and turbidity becomes significant whereby that is not preferred. Accordingly, it is more preferred to be 0.02 to 4 part(s) by weight, and further more preferred to be 0.03 to 3 part(s) by weight. Moreover, it is not excluded that, besides the (meth)acrylate copolymer, an antibacterial material or the like is further added to a pellet-shaped composition for medical use.

In the present invention, adding amount of a plasticizer is preferred to be 1 to 120 part(s) by weight to 100 parts by weight of a polyvinyl chloride resin. When the amount of a plasticizer is too small, there is a possibility of deterioration of flexibility which is necessary for a molded product prepared by a melt molding of a pellet-shape composition for medical use or particularly for a tube for medical use. Moreover, when the amount of a plasticizer is too much, there is a risk that flexibility becomes too high whereby kink is resulted during its use. Accordingly, the amount of a plasticizer is more preferred to be 10 to 115 parts by weight, and further more preferred to be 30 to 110 parts by weight.

In the present invention, number-average polymerization degree of a polyvinyl chloride resin is preferred to be 700 to 3000. When number-average polymerization degree is within this range, the molded product can achieve a good durability. When it is too low, durability of the molded product is insufficient. When it is too high, intramolecular and/or intermolecular hydrogen bond(s) of polyvinyl chloride become(s) strong and uniform mixing with a plasticizer and a (meth)acrylate copolymer becomes difficult whereby it is difficult to achieve a good appearance. Accordingly, it is more preferred to be 800 to 2800, and further more preferred to be 900 to 2600.

The present inventors investigated the relation between the transparency of a pellet-shaped composition for medical use and the number-average molecular weight of a (meth)acrylate copolymer, the repeating unit of polyethylene glycol or the molar ratio of a hydrophilic acrylate. The transparency is evaluated in terms of glossiness which will be mentioned later. The result thereof is schematically shown in Fig. 2. It has been found that, when molar ratio of a hydrophilic (meth)acrylate is shown in an abscissa while number-average molecular weigh of a (meth)acrylate copolymer is shown in an ordinate, transparency becomes good in a tube which is molded using a pellet-shaped composition for medical use comprising a (meth)acrylate copolymer wherein the molar ratio of a hydrophilic (meth)acrylate is 45 molar % or less, the number-average molecular weight of a (meth)acrylate copolymer is 50,000 or less and the polyethylene glycol repeating units are 4 or less. In a tube having outer diameter of 9.2 to 15 mm, only the case wherein glossiness is 30 or more can be judged to be good in terms of transparency.

Then the relation between the antithrombotic property and a pellet-shaped composition for medical use was investigated in the same manner. The result is shown in Fig. 3. The antithrombotic property of a pellet-shaped composition for medical use was good when a hydrophilic (meth) acrylate was made 20 molar % or more, number-average molecular weight of a (meth)acrylate copolymer was made 7,000 or more and a polyethylene glycol repeating units were made 2 or more. The antithrombotic property was confirmed by means of evaluation of a wettability which will be mentioned later. When it was combined with the above-mentioned result for the transparency, it was noted to be necessary for achieving both of the transparency and the antithrombotic property in a pellet-shaped composition for medical use that hydrophilic (meth)acrylate units are made 20 to 45 molar %, number-average molecular weight of a (meth)acrylate copolymer is made 7,000 to 50,000 and polyethylene glycol repeating units are made 2 to 4.

In a (meth)acrylate copolymer prepared by copolymerization of a hydrophobic (meth)acrylate unit with a hydrophilic (meth)acrylate unit in the present invention, hydrophilicity and hydrophobicity are well-balanced whereby the copolymer can be suitably used as an antithrombotic material. It is also possible in the present invention that two or more kinds of (meth)acrylate copolymers are mixed and used.

In a specific method for kneading a polyvinyl chloride resin, a plasticizer and a (meth)acrylate copolymer in the present invention, the kneading is carried out at 20 to 200 rpm for 0.2 to 5 hour (s) under heating at 100 to 180°C. After that, the above kneaded mixture is provided to a hopper of a biaxial extruder (such as PCM-30 manufactured by Ikegai) having a cylinder temperature adjusted to 150 to 200°C and melted with a screw revolution of 80 to 120 rpm and the melted resin is extruded in an extrusion speed of 200 to 400 g/minute. It is then pulled at the rate of 10 to 60 m/minute in a water tank having a length of 1 to 20 m and an inner temperature of 10 to 20°C to give a strand having the outer diameter of 1 to 5 mm. The strand is then cut using a cutter (such as FCMini-6/N manufactured by Hoshi Plastics) so as to make its length 1 to 5 mm followed by cooling to give a pellet-shaped composition for medical use.

As hereunder, a method for molding a tube for medical use using the pellet-shaped composition for medical use of the present invention will be illustrated in detail. Fig. 4 is a schematic drawing of an extrusion molding machine for manufacturing a tube for medical use of the present invention. The machine is equipped with a supplying part (hopper) for supplying the pellets (a starting material) of a pellet-shaped composition for medical use, a cylinder for forming a route wherein the starting material supplied from the supplying part is dissolved and conveyed, a screw which is arranged

in the inner area of the cylinder for dissolving and conveying the material, and a screw driving part which drives the screw. A die for adjusting the cross-sectional shape of the tube is further attached to an outlet side of the cylinder. The extrusion molding machine may be also in such a constitution that a supplying/dissolving/conveying means part (supplying part, cylinder and screw) and a quantitatively discharging part (gear pump and a housing part therefor) may be united or may be also in such a constitution that a quantitatively discharging part is attached to a supplying/kneading/conveying means part.

In Fig. 4, although a screw is in a uniaxial constitution, a biaxial constitution may be also applied thereto. Further, a supplying part may be equipped with a quantitatively supplying device. Still further, a screw driving part may be constituted by an appropriate combination of publicly known motor, driving mechanism, means for changing the rotation speed (mechanism for speed reduction), means for changing the torque, etc. Furthermore, a device for adjusting the temperature in the inner area of a cylinder may be provided.

In the present invention, the resulting pellets are poured from a material supplying part of an extrusion molding machine, dissolved by a screw in a cylinder and extruded from a die making into a ring shape at the resin temperature of 180 to 195°C. When the temperature is too low at that time, transparency of the tube may lower. Accordingly, the resin temperature is more preferred to be 182°C or higher. When the resin temperature is too high, there is a risk that polyvinyl chloride is oxidized and a double bond is generated in main chain of the polymer whereby the tube is colored. Accordingly, the resin temperature is more preferred to be 192°C or lower. Incidentally, the term reading resin temperature used herein stands for the temperature of a melted resin measured at the area which is near the outlet of a cylinder.

The melted resin which is dissolved and conveyed by a screw is extruded from a die provided in the outlet side of a cylinder. At that time, the die used therefor is preferred to be a straight die of a spiral type. Since a material dissolved at relatively low temperature is used in the present invention, spider mark is apt to be generated. Therefore, it is preferred to use a spiral die.

In the present invention, rotation speed of a screw is preferred to be 30 to 70 rpm. When the rotation speed of the screw is too low, shearing stress becomes small whereby dissolution deficiency is apt to happen. Particular in the case of dissolution at relatively low temperature as in the present invention, it is preferred that the rotation speed of a screw is set a bit higher. Although it is to be set a bit higher, it should not be set unlimitedly high. When the relation between the viscosity of the melted resin and the torque of the motor rotating a screw and influence of a rise in the resin temperature by heat of friction, etc. are taken into consideration, more preferred range thereof is 35 to 65 rpm.

In the present invention, it is preferred to use a screw having the compression ratio of 2.5 to 6.0. When the compression ratio is too small or too large, the shearing stress applied to the material flowing in a cylinder is not homogenized whereby dissolution deficiency may happen.

The melted resin extruded from a die is subjected to sizing into a predetermined size in a vacuum water tank and cooled down to around the room temperature by a cooling water tank. The resulting tube is rolled using a winding machine. At that time, a size measuring device or a printer may be provided between the cooling water tank and the wining machine. Temperature of cooling water is preferred to be 5 to 30°C. When the temperature is lower than 30°C, it is preferred since the adjustment of inner and outer diameters is easy while, when it is higher than 5°C, it is preferred since water is not frozen. Incidentally, degree of vacuum is from (ordinary pressure) - 20 to -1 kPa.

In the present invention, the pulling speed is preferred to be 4 to 40 m/min. When it is higher than 4 m/min, productivity increased whereby it is preferred while, when it is lower than 40 m/min, sufficient transparency can be achieved whereby it is preferred.

When the pellet-shaped composition for medical use according to the present invention is molded into a tube or the like, wettability of the surface is improved due to a hydrophilic effect of methoxy polyethylene glycol (meth)acrylate in a (meth)acrylate copolymer. As a result of an improvement in the wettability, it has been confirmed that an antithrombotic property such as suppression of platelet adhesion or blood coagulation can be imparted. When the ratio of the methoxy polyethylene glycol (meth)acrylate unit is raised too much, the wettability increases but, on the other hand, elution into blood becomes significant whereby it becomes difficult to maintain the antithrombotic property for a long period.

As to a method for evaluating the wettability of the pellet-shaped composition for medical use according to the present invention, a conventional method by means of contact angle is exemplified. However, although such a method is advantageous in a sample of a flat plate shape, it cannot be applied as it is for the evaluation of a tube comprising a pellet-shaped composition for medical use since the contacting surface is curved. Therefore, the present inventors have been conducted intensive investigations for a method of evaluating the tubular sample. Thus, when one drop of water in a predetermined volume is dropped into an inner area (concave area) of a tube which is cut into one half in a longitudinal direction and the water drop is then moved both forwardly and backwardly in the longitudinal direction of the tube, the water drop is extended in the front and back directions depending upon the wettability of the surface. According to this method, the wettability to the curved surface can be advantageously evaluated. With regard to width of a water drop for which the wettability is judged to be appropriate, although it depends upon the inner diameter of a tube used for the evaluation, it is preferred to be 0.9 to 2.0 cm. When the water drop width is within this range, it can be regarded that wettability is improved as compared with a composition which is solely composed of polyvinyl chloride and plasticizer.

When the water drop width is too small, it is not preferred since no antithrombotic property is achieved thereby while, when it is too large, it is not preferred since there is a risk of elution into blood. Thus, the range of 1.0 to 1.5 cm is more preferred.

It is necessary in the present invention that the amount of water drop used for the evaluation of wettability is appropriately selected depending upon the inner diameter of a tube. This is because, when the amount of water drop is too much or too small, it is difficult to evaluate the difference among the samples. To be more specific, in the case of a tube having inner diameter of 6.0 to 6.7 mm, amount of water drop is preferred to be 0.07 mL and, similarly, in the case of 6.8 to 8.5 mm, the amount is preferred to be 0.085 mL and, in the case of 8.6 to 14.0 mm, the amount is preferred to be 0.1 mL. When the amount of water drop is within such a range, the wettability can be strictly evaluated.

As to an inner diameter of a tube used for the evaluation of wettability in the present invention, it is preferred to be 5 mm or more. When it is within such a range, it is possible to secure sufficient area wherein a water drop can touch the inner surface whereby the difference in the wettability among the samples is apt to be easily evaluated. When the inner diameter is too small, a work for cutting the tube in a longitudinal direction becomes difficult whereby it is not preferred. Thus, the inner diameter is more preferred to be 5.5 mm or longer, and further more preferred to be 6 mm or longer.

In the tube for medical use according to the present invention, there is a certain relationship between the transparency of the body and the gloss. Thus, the higher transparency the body has, the less diffused reflection of the incident light occurs. Accordingly, the intensity of the reflected light or the glossiness becomes high. When the glossiness is high, diffused reflection of the light on the outer surface of a tube is small whereby visibility of the inner area of the tube is improved. However, even in a tube having the transparency in the similar degree, curvature changes when the outer diameter thereof changes whereby the glossiness does not become the same value. The smaller curvature the tube has, the more intensity of the reflected light occurs. It is preferred that the glossiness of the outer surface of a tube is measured by a glossimeter in accordance with a method mentioned in JIS Z 8741 (1997) under the conditions wherein incident/reflected angle (light-receiving angle) is 60°, measuring area is 3 mm × 3 mm and light-receiving part is 3. 02 mm × 1.51 mm. When the measuring conditions as such are used, measurement of glossiness is possible even in a tube having small outer diameter due to small measuring area. When outer diameter of a tube is 7 to 9 mm, the glossiness is preferred to be 25 or more. When outer diameter of a tube is 9.2 to 15 mm, the glossiness is preferred to be 30 or more, and more preferred to be 35 or more. When it is 17 to 19 mm, the glossiness is preferred to be 40 or more. When the glossiness is within such a range, good transparency can be achieved whereby visibility of the inner area of a tube can be secured.

In the present invention, number-average molecular weight (Mn) is defined by the following formula when molecules having a molecular weight of Mi are present in the numbers of Ni in a polymer (cf. for example, POLYMER CHEMISTRY, OXFORD UNIVERSITY PRESS, p.36 (1999)).

$$M_n = \frac{\text{Total weight of system}}{\text{Number of molecules in system}} = \frac{\sum M_i N_i}{\sum N_i}$$

The number-average polymerization degree of the present invention can be calculated from the relation that "(number-average molecular weight) = (molecular weight of monomer) × (number-average polymerization degree)". It can be used as an index for the molecular weight of polyvinyl chloride resin the same as in the case of number-average molecular weight.

Inner diameter of a tube for medical use is preferred to be 0.1 to 30 mm. When the inner diameter is too small, there is a possibility that thrombus is apt to be generated or kink is resulted during the use. When the inner diameter of a tube is too large, handling becomes difficult or control of flow rate of blood becomes difficult whereby that is not preferred. Accordingly, the inner diameter of a tube is more preferred to be 1 to 25 mm, and further more preferred to be 2 to 20 mm.

Although the thickness of a tube is not particularly limited, it is preferred to be 0 . 2 to 5 mm in view of the intention of the present invention. When the thickness is too thin, strength may become low. When the thickness is too thick, there is a possibility that flexibility of a tube is not sufficient or visibility of the inner area lowers. Accordingly, the thickness of a tube is more preferred to be 0.6 to 4 mm, and further more preferred to be 1 to 3 mm.

As to a plasticizer for the present invention, it is preferred to use di-2-ethylhexyl phthalate (DOP), tri-di-2-ethylhexyl trimellitate (TOTM) or diisononyl cyclohexane-1,2-dicarboxylate (DINCH). In view of plasticizing efficiency and cost, it is more preferred to use DOP.

Examples

As hereunder, the present invention will be more specifically illustrated by referring to Examples but the present invention is not limited to those Examples.

(Evaluation of wetting property)

As shown in Fig. 5, the resulting tube was cut in 3 cm length and further cut horizontally in the longitudinal direction so that the cross section thereof becomes semicircular so as to make into a gutter shape. One drop of water was dropped onto the center of the gutter-shaped sample. Amounts of the dropped water were made 0.070 mL, 0.085 mL and 0.1 mL when the inner diameters of a tube were 6.0 to 6.7 mm, 6.8 to 8.5 mm and 8.6 to 14.0 mm, respectively. The sample was inclined in the longitudinal direction, the water drop was carefully moved in forward and backward directions within about 1 cm for one time each so as not to spill. Then, it was returned to the predetermined position and the water drop width was measured. The same operation was conducted for four times for each sample and mean value thereof was determined. When the water drop width was 1.0 cm to 1.5 cm, 0.9 cm to less than 1.0 cm and less than 0.9 cm, it was judged as "very good", "good" and "not good", respectively. The case of "very good" or "good" is judged to be "passing the test".

(Measurement of number-average molecular weight)

A (meth) acrylate copolymer (15 mg) was dissolved in 3 mL of a mobile phase for the GPC measurement and filtered through a hydrophilic PTFE (Millex-LH of Nippon Millipore) of 0.45 μm. The GPC measurement was conducted in following conditions. Thus, a 510 high pressure pump, a 717 plus automated infusion apparatus (Nippon Water) and a measuring apparatus of RI-101 (Showa Denko) were used; the column was PLgel 5 μMiXED-D (600 × 7.5 mm) (Polymer Laboratories) ; temperature of the column was set at ambient temperature; and tetrahydrofuran (THF) supplemented with 0.03% by weight of dibutyl hydroxytoluene (BHT) was used as a mobile phase. Detection was conducted by RI and 50 μL was infused. Calibration of molecular weight was conducted using a monodispersed PMMA (Easi Cal: Polymer Laboratories).

(Measurement of copolymerized composition ratio)

A (meth) acrylate copolymer (50 mg) was added into a test tube for NMR (Standard: N-5; manufactured by Nippon Seimitsu Kagaku) using a Pasteur pipette. Then, 0.7 mL of heavy chloroform (manufactured by Wako Pure Chemicals) was added thereto and well mixed. Then, the tube was covered with a cap for specimen (Standard: NC-5; manufactured by Nippon Seimitsu Kagaku). The copolymerized composition ratio was calculated by conducting a 1H NMR measurement at room temperature using GEMINI-200 of Varian.

(Measurement of inner and outer diameters of tube)

Inner and outer diameters of a tube were measured using a projector (V-12B manufactured by Nikon). Thus, a tube was cut into 3-mm thickness using a hose cutter (HC03) to prepare a sample. Magnification was set 10-fold. A stage was moved so that lower right area of the outer surface of a sample contacted X and Y axes by checking the projection to reset the X and Y coordinates and then the stage was moved to the place where the upper left area of the outer surface of the sample contacted the X and Y axes whereupon the mean value A of X and Y coordinates was taken. Then the stage was further moved so that X and Y axes contacted the upper right area of the outer surface of the sample, the values of the X and Y coordinates were reset, then X and Y coordinates were moved so as to contact the lower left area of the outer surface of the sample, and the mean value B of the values of X and Y coordinates was taken. The mean value C of A and B was adopted as the value of an outer diameter. Similar measurement was also conducted for the inner surface as well and the value of inner diameter was calculated.

(Measurement of glossiness)

Measurement of glossiness of the outer surface of a tube was conducted in accordance with a method mentioned in JIS Z 8741(1997) using a digital precise glossimeter GM-26D manufactured by KK Murakami Color Technology Laboratory. A sample which was cut into 40 mm length was fixed on a sample stand (100 × 120 mm) which had been subjected to a V-shaped grooving process for enabling fixation of a cylindrical sample. Glossiness was measured at the incident/reflected angle (light-receiving angle) of 60°. Measuring area and light-receiving part were made 3 × 3 mm and 3.02 × 1.51 mm, respectively. When the glossiness in the outer diameter of a tube in this Example was 35 or more, 30

to less than 35 and less than 30, they were evaluated as "very good", "good" and "not good", respectively. The case of "very good" or "good" is judged as "passing the test".

(Synthesis of (meth)acrylate copolymer 1)

To a reactor being able to be stirred and equipped with a refluxing tower were added 17.5 g of methoxytriethylene glycol acrylate (MTEGA) (manufactured by Shin Nakamura Kagaku Kogyo), 27.4 g of 2-ethylhexyl acrylate (EHA) (manufactured by Tokyo Kasei Kogyo), 0.0447 g of azobisisobutyronitrile (AIBN) (manufactured by Wako Pure Chemical Industries) and 178.9 g of ethanol (manufactured by Tokyo Kasei Kogyo) followed by subjecting to a polymerization reaction at 80°C for 20 hours. In the meanwhile, the inner area of the reactor was previously substituted with nitrogen and, during the polymerization reaction, bubbling with nitrogen was continued. After completion of the polymerization reaction, the solvent for polymerization was removed by evaporation for four days under the condition of 60°C and 1 Torr to give a crude (meth) acrylate copolymer. The resulting crude (meth) acrylate copolymer (2 g) was dissolved in 2 g of tetrahydrofuran and dropped into 20 g of a poor solvent (comprising methanol and water in a ratio by weight of 80/20) under stirring using a Pasteur pipette. An operation comprising that the precipitate was recovered by means of decantation, dissolved again by addition of tetrahydrofuran in the same weight and dropped into a poor solvent was repeated twice and, after that, drying *in vacuo* was carried out for four days under the vacuum condition of 0.1 Torr at 60°C to give a purified product 1.

(Synthesis of (meth)acrylate copolymer 2)

To a reactor being able to be stirred and equipped with a refluxing tower were added 27.6 g of MTEGA (manufactured by Shin Nakamura Kagaku Kogyo), 28.4 g of EHA (manufactured by Tokyo Kasei Kogyo), 0.0543 g of AIBN (manufactured by Wako Pure Chemical Industries) and 115.4 g of ethanol (manufactured by Tokyo Kasei Kogyo) followed by subjecting to a polymerization reaction at 80°C for 20 hours. After finishing the polymerization reaction, the same operation as in "Synthesis of (meth)acrylate copolymer 1" was conducted to give a crude (meth)acrylate copolymer. The resulting crude (meth)acrylate copolymer was subjected to the same treatment as in "Synthesis of (meth)acrylate copolymer 1" to give a purified product 2.

(Synthesis of (meth)acrylate copolymer 3)

To a reactor being able to be stirred and equipped with a refluxing tower were added 15.9 g of MTEGA (manufactured by Shin Nakamura Kagaku Kogyo), 40.1 g of EHA (manufactured by Tokyo Kasei Kogyo), 0. 0543 g of AIBN (manufactured by Wako Pure Chemical Industries) and 53.6 g of ethanol (manufactured by Tokyo Kasei Kogyo) followed by subjecting to a polymerization reaction at 80°C for 20 hours. After finishing the polymerization reaction, the same operation as in "Synthesis of (meth)acrylate copolymer 1" was conducted to give a crude (meth)acrylate copolymer. The resulting crude (meth)acrylate copolymer was subjected to the same treatment as in "Synthesis of (meth)acrylate copolymer 1" to give a purified product 3.

(Synthesis of (meth)acrylate copolymer 4)

To a reactor being able to be stirred and equipped with a refluxing tower were added 15.7 g of MTEGA (manufactured by Shin Nakamura Kagaku Kogyo), 40.3 g of lauryl acrylate (LA) (manufactured by Shin Nakamura Kagaku Kogyo), 0.0543 g of AIBN (manufactured by Wako Pure Chemical Industries) and 35.9 g of ethanol (manufactured by Tokyo Kasei Kogyo) followed by subjecting to a polymerization reaction at 80°C for 20 hours. After finishing the polymerization reaction, the same operation as in "Synthesis of (meth)acrylate copolymer 1" was conducted to give a crude (meth)acrylate copolymer. The resulting crude (meth)acrylate copolymer was subjected to the same treatment as in "Synthesis of (meth)acrylate copolymer 1" to give a purified product 4.

(Synthesis of (meth)acrylate copolymer 5)

To a reactor being able to be stirred and equipped with a refluxing tower were added 21.8 g of MTEGA (manufactured by Shin Nakamura Kagaku Kogyo), 34.1 g of EHA (manufactured by Tokyo Kasei Kogyo), 0. 0543 g of AIBN (manufactured by Wako Pure Chemical Industries) and 30.9 g of ethanol (manufactured by Tokyo Kasei Kogyo) followed by subjecting to a polymerization reaction at 80°C for 20 hours. After finishing the polymerization reaction, the same operation as in "Synthesis of (meth)acrylate copolymer 1" was conducted to give a crude (meth) acrylate copolymer. The resulting crude (meth)acrylate copolymer was subjected to the same treatment as in "Synthesis of (meth) acrylate copolymer 1" to give a purified 5.

(Synthesis of (meth)acrylate copolymer 6)

To a reactor being able to be stirred and equipped with a refluxing tower were added 18.9 g of MTEGA (manufactured by Shin Nakamura Kagaku Kogyo), 27.7 g of 2-ethylhexyl acrylate (EHA) (manufactured by Tokyo Kasei Kogyo), 0.0543 g of AIBN (manufactured by Wako Pure Chemical Industries), 3.1 g of polydimethylsiloxane methacrylate (PDMSA) (manufactured by Gelest, catalog number: MCR-M11) and 89.5 g of ethanol (manufactured by Tokyo Kasei Kogyo) followed by subjecting to a polymerization reaction at 80°C for 20 hours. After finishing the polymerization reaction, the same operation as in "Synthesis of (meth) acrylate copolymer 1" was conducted to give a crude (meth)acrylate copolymer. The resulting crude (meth) acrylate copolymer was subjected to the same treatment as in "Synthesis of (meth) acrylate copolymer 1" to give a purified product 6.

(Synthesis of (meth) acrylate copolymer 7)

To a reactor being able to be stirred and equipped with a refluxing tower were added 15.9 g of MTEGA (manufactured by Shin Nakamura Kagaku Kogyo), 70.8 g of stearyl acrylate (manufactured by Wako Pure Chemical Industries), 0.0543 g of AIBN (manufactured by Wako Pure Chemical Industries) and 86.7 g of ethanol (manufactured by Tokyo Kasei Kogyo) followed by subjecting to a polymerization reaction at 80°C for 20 hours. After finishing the polymerization reaction, the same operation as in "Synthesis of (meth) acrylate copolymer 1" was conducted to give a crude (meth)acrylate copolymer. The resulting crude (meth)acrylate copolymer was subjected to the same treatment as in "Synthesis of (meth) acrylate copolymer 1" to give a purified product 7.

(Synthesis of (meth)acrylate copolymer 8)

To a reactor being able to be stirred and equipped with a refluxing tower were added 15.9 g of MTEGA (manufactured by Shin Nakamura Kagaku Kogyo), 28.0 g of butyl acrylate (manufactured by Wako Pure Chemical Industries), 0.0543 g of AIBN (manufactured by Wako Pure Chemical Industries) and 43.8 g of ethanol (manufactured by Tokyo Kasei Kogyo) followed by subjecting to a polymerization reaction at 80°C for 20 hours. After finishing the polymerization reaction, the same operation as in "Synthesis of (meth)acrylate copolymer 1" was conducted to give a crude (meth)acrylate copolymer. The resulting crude (meth)acrylate copolymer was subjected to the same treatment as in "Synthesis of (meth)acrylate copolymer 1" to give a purified product 8.

(Synthesis of (meth)acrylate copolymer 9)

To a reactor being able to be stirred and equipped with a refluxing tower were added 6.3 g of MTEGA (manufactured by Shin Nakamura Kagaku Kogyo), 48.2 g of EHA (manufactured by Tokyo Kasei Kogyo), 0. 0543 g of AIBN (manufactured by Wako Pure Chemical Industries) and 54.5 g of ethanol (manufactured by Tokyo Kasei Kogyo) followed by subjecting to a polymerization reaction at 80°C for 20 hours. After finishing the polymerization reaction, the same operation as in "Synthesis of (meth)acrylate copolymer 1" was conducted to give a crude (meth)acrylate copolymer. The resulting crude (meth)acrylate copolymer was subjected to the same treatment as in "Synthesis of (meth)acrylate copolymer 1" to give a purified product 9.

(Synthesis of (meth)acrylate copolymer 10)

To a reactor being able to be stirred and equipped with a refluxing tower were added 31.7 g of MTEGA (manufactured by Shin Nakamura Kagaku Kogyo), 26.8 g of EHA (manufactured by Tokyo Kasei Kogyo), 0. 0543 g of AIBN (manufactured by Wako Pure Chemical Industries) and 58.5 g of ethanol (manufactured by Tokyo Kasei Kogyo) followed by subjecting to a polymerization reaction at 80°C for 20 hours. After finishing the polymerization reaction, the same operation as in "Synthesis of (meth)acrylate copolymer 1" was conducted to give a crude (meth)acrylate copolymer. The resulting crude (meth)acrylate copolymer was subjected to the same treatment as in "Synthesis of (meth)acrylate copolymer 1" to give a purified product 10.

(Synthesis of (meth)acrylate copolymer 11)

To a reactor being able to be stirred and equipped with a refluxing tower were added 35.1 g of methoxynonaethyleneglycol acrylate (manufactured by Shin Nakamura Kagaku Kogyo), 40.1 g of EHA (manufactured by Tokyo Kasei Kogyo), 0.0543 g of AIBN (manufactured by Wako Pure Chemical Industries) and 75.2 g of ethanol (manufactured by Tokyo Kasei Kogyo) followed by subjecting to a polymerization reaction at 80°C for 20 hours. After finishing the polymerization reaction, the same operation as in "Synthesis of (meth)acrylate copolymer 1" was conducted to give a crude

(meth)acrylate copolymer. The resulting crude (meth)acrylate copolymer was subjected to the same treatment as in "Synthesis of (meth)acrylate copolymer 1" to give a purified product 11.

(Synthesis of (meth)acrylate copolymer 12)

To a reactor being able to be stirred and equipped with a refluxing tower were added 9.5 g of 2-methoxyethyl acrylate (manufactured by Wako Pure Chemical Industries), 40.1 g of EHA (manufactured by Tokyo Kasei Kogyo), 0.0543 g of AIBN (manufactured by Wako Pure Chemical Industries) and 49.6 g of ethanol (manufactured by Tokyo Kasei Kogyo) followed by subjecting to a polymerization reaction at 80°C for 20 hours. After finishing the polymerization reaction, the same operation as in "Synthesis of (meth)acrylate copolymer 1" was conducted to give a crude (meth)acrylate copolymer. The resulting crude (meth)acrylate copolymer was subjected to the same treatment as in "Synthesis of (meth)acrylate copolymer 1" to give a purified product 12.

(Synthesis of (meth)acrylate copolymer 13)

To a reactor being able to be stirred and equipped with a refluxing tower were added 56.0 g of EHA (manufactured by Tokyo Kasei Kogyo), 0. 0543 g of AIBN (manufactured by Wako Pure Chemical Industries) and 56.0 g of ethanol (manufactured by Tokyo Kasei Kogyo) followed by subjecting to a polymerization reaction at 80°C for 20 hours. After finishing the polymerization reaction, the same operation as in "Synthesis of (meth)acrylate copolymer 1" was conducted to give a crude (meth)acrylate copolymer. The resulting crude (meth)acrylate copolymer was subjected to the same treatment as in "Synthesis of (meth)acrylate copolymer 1" to give a purified product 13.

(Synthesis of (meth)acrylate copolymer 14)

To a reactor being able to be stirred and equipped with a refluxing tower were added 15.9 g of MTEGA (manufactured by Shin Nakamura Kagaku Kogyo), 40.1 g of EHA (manufactured by Tokyo Kasei Kogyo), 0. 0543 g of AIBN (manufactured by Wako Pure Chemical Industries) and 20.0 g of ethanol (manufactured by Tokyo Kasei Kogyo) followed by subjecting to a polymerization reaction at 80°C for 20 hours. After finishing the polymerization reaction, the same operation as in "Synthesis of (meth)acrylate copolymer 1" was conducted to give a crude (meth)acrylate copolymer. The resulting crude (meth)acrylate copolymer was subjected to the same treatment as in "Synthesis of (meth)acrylate copolymer 1" to give a purified product 14.

(Synthesis of (meth)acrylate copolymer 15)

To a reactor being able to be stirred and equipped with a refluxing tower were added 15.9 g of MTEGA (manufactured by Shin Nakamura Kagaku Kogyo), 40.1 g of EHA (manufactured by Tokyo Kasei Kogyo), 0.0543 g of AIBN (manufactured by Wako Pure Chemical Industries) and 350.0 g of ethanol (manufactured by Tokyo Kasei Kogyo) followed by subjecting to a polymerization reaction at 80°C for 20 hours. After finishing the polymerization reaction, the same operation as in "Synthesis of (meth)acrylate copolymer 1" was conducted to give a crude (meth)acrylate copolymer. The resulting crude (meth)acrylate copolymer was subjected to the same treatment as in "Synthesis of (meth)acrylate copolymer 1" to give a purified product 15.

(Example 1)

A polyvinyl chloride resin (number-average polymerization degree: 1,000) (10 kg), 1.0 kg of DOP and 5 g of a (meth) acrylate copolymer 1 were charged and kneaded at 155°C for 3 hours. Then, pellets were prepared at 160°C and 100 rpm using a biaxial extruder. The resulting pellets were supplied to a supplying part (hopper) and transferred into a cylinder. Screw having compression ratio of 4 was rotated at 56.0 rpm and the pellets were transferred to the front area of the cylinder by melting them at the cylinder temperature of 170°C. The resulting melted resin was extruded from spiral dies of the outlet area of the cylinder. Temperature of the resin at the outlet area of the cylinder at that time was 188°C. The melted resin extruded therefrom was passed through a vacuum water tank having the temperature of 15°C and then cooled down to nearly the room temperature using the cooling water tank of the same temperature. Degree of vacuum of the vacuum water tank at that time was made -7.0 kPa. The resulting tube was wound around a winding machine at a pulling speed of 10 m/min.

(Example 2)

A polyvinyl chloride resin (number-average polymerization degree: 2,000) (10 kg), 3.0 kg of TOTM and 10 g

of a (meth)acrylate copolymer 2 were charged and kneaded at 155°C for 3 hours. They were further subjected to a treatment at 160°C and 100 rpm using a biaxial extruder to prepare pellets. The resulting pellets were treated in the same manner as in Example 1 to prepare a tube.

(Example 3)

A polyvinyl chloride resin (number-average polymerization degree: 2,500) (10 kg), 5.0 kg of DOP and 100 g of a (meth) acrylate copolymer 3 were charged and kneaded at 155°C for 3 hours. They were further subjected to a treatment at 160°C and 100 rpm using a biaxial extruder to prepare pellets. The resulting pellets were treated in the same manner as in Example 1 to prepare a tube.

(Example 4)

A polyvinyl chloride resin (number-average polymerization degree: 2,500) (10 kg), 11.0 kg of DOP and 300 g of a (meth)acrylate copolymer 4 were charged and kneaded at 155°C for 3 hours. They were further subjected to a treatment at 160°C and 100 rpm using a biaxial extruder to prepare pellets. The resulting pellets were treated in the same manner as in Example 1 to prepare a tube.

(Example 5)

A polyvinyl chloride resin (number-average polymerization degree: 2, 500) (10 kg), 11.0 kg of DINCH and 100 g of a (meth)acrylate copolymer 5 were charged and kneaded at 155°C for 3 hours. They were further subjected to a treatment at 160°C and 100 rpm using a biaxial extruder to prepare pellets. The resulting pellets were treated in the same manner as in Example 1 to prepare a tube.

(Example 6)

A polyvinyl chloride resin (number-average polymerization degree: 2,500) (10 kg), 5 kg of DOP and 100 g of a (meth) acrylate copolymer 6 were charged and kneaded at 155°C for 3 hours. They were further subj ected to a treatment at 160°C and 100 rpm using a biaxial extruder to prepare pellets. The resulting pellets were treated in the same manner as in Example 1 to prepare a tube.

(Example 7)

A polyvinyl chloride resin (number-average polymerization degree: 2,500) (10 kg), 5 kg of DOP and 100 g of a (meth) acrylate copolymer 7 were charged and kneaded at 155°C for 3 hours. They were further subjected to a treatment at 160°C and 100 rpm using a biaxial extruder to prepare pellets. The resulting pellets were treated in the same manner as in Example 1 to prepare a tube.

In the tube prepared in this Example, its water drop width was "good" probably due to the fact that carbon atom number of the alkyl (meth)acrylate was big.

(Example 8)

A polyvinyl chloride resin (number-average polymerization degree: 2,500) (10 kg), 5 kg of DOP and 100 g of a (meth) acrylate copolymer 8 were charged and kneaded at 155°C for 3 hours. They were further subj ected to a treatment at 160°C and 100 rpm using a biaxial extruder to prepare pellets. The resulting pellets were treated in the same manner as in Example 1 to prepare a tube.

In the tube prepared in this Example, its glossiness was "good" probably due to the fact that carbon atom number of the alkyl (meth)acrylate was small.

(Example 9)

A polyvinyl chloride resin (number-average polymerization degree: 2,500) (10 kg), 5 kg of DOP and 100 g of a (meth) acrylate copolymer 9 were charged and kneaded at 155°C for 3 hours. They were further subj ected to a treatment at 160°C and 100 rpm using a biaxial extruder to prepare pellets. The resulting pellets were treated in the same manner as in Example 1 to prepare a tube.

In the tube prepared in this Example, its water drop width was "good" probably due to the fact that molar ratio of the hydrophilic (meth)acrylate was small.

(Example 10)

A polyvinyl chloride resin (number-average polymerization degree: 2,500) (10 kg), 5 kg of DOP and 100 g of a (meth)acrylate copolymer 10 were charged and kneaded at 155°C for 3 hours. They were further subjected to a treatment at 160°C and 100 rpm using a biaxial extruder to prepare pellets. The resulting pellets were treated in the same manner as in Example 1 to prepare a tube.

In the tube prepared in this Example, its glossiness was "good" probably due to the fact that molar ratio of the hydrophilic (meth)acrylate was big.

(Example 11)

A polyvinyl chloride resin (number-average polymerization degree: 2,500) (10 kg), 5 kg of DOP and 100 g of a (meth)acrylate copolymer 11 were charged and kneaded at 155°C for 3 hours. They were further subjected to a treatment at 160°C and 100 rpm using a biaxial extruder to prepare pellets. The resulting pellets were treated in the same manner as in Example 1 to prepare a tube.

In the tube prepared in this Example, its glossiness was "good" probably due to the fact that number of the polyethylene glycol repeating unit was big.

(Example 12)

A polyvinyl chloride resin (number-average polymerization degree: 2,500) (10 kg), 5 kg of DOP and 100 g of a (meth)acrylate copolymer 12 were charged and kneaded at 155°C for 3 hours. They were further subjected to a treatment at 160°C and 100 rpm using a biaxial extruder to prepare pellets. The resulting pellets were treated in the same manner as in Example 1 to prepare a tube.

In the tube prepared in this Example, its water drop width was "good" probably due to the fact that number of the polyethylene glycol repeating unit was small.

(Comparative Example 1)

A polyvinyl chloride resin (number-average polymerization degree: 2,500) (10 kg), 5 kg of DOP and 100 g of a (meth)acrylate copolymer 13 were charged and kneaded at 155°C for 3 hours. They were further subjected to a treatment at 160°C and 100 rpm using a biaxial extruder to prepare pellets. The resulting pellets were treated in the same manner as in Example 1 to prepare a tube.

In the tube prepared in this Comparative Example, its water drop width was "not good" probably due to the fact that no hydrophilic (meth)acrylate was used.

(Comparative Example 2)

A polyvinyl chloride resin (number-average polymerization degree: 2,500) (10 kg), 5 kg of DOP and 100 g of a (meth)acrylate copolymer 14 were charged and kneaded at 155°C for 3 hours. They were further subjected to a treatment at 160°C and 100 rpm using a biaxial extruder to prepare pellets. The resulting pellets were treated in the same manner as in Example 1 to prepare a tube.

In the tube prepared in this Comparative Example, its glossiness was "not good" probably due to the fact that molecular weight of the (meth) acrylate copolymer was too high.

(Comparative Example 3)

A polyvinyl chloride resin (number-average polymerization degree: 2,500) (10 kg), 5 kg of DOP and 600 g of a (meth) acrylate copolymer 3 were charged and kneaded at 155°C for 3 hours. It was further attempted to prepare pellets at 160°C and 100 rpm using a biaxial extruder but no strand can be prepared and no pellet can be formed probably due to the fact that adding amount of the (meth) acrylate copolymer was too much.

(Comparative Example 4)

A polyvinyl chloride resin (number-average polymerization degree: 2,500) (10 kg), 5.0 kg of DOP and 0.1 g of a (meth) acrylate copolymer 3 were charged and kneaded at 155°C for 3 hours. They were further subjected to a treatment at 160°C and 100 rpm using a biaxial extruder to prepare pellets. The resulting pellets were treated in the same manner as in Example 1 to prepare a tube.

In the tube prepared in this Comparative Example, its water drop width was "not good" probably due to the fact that adding amount of the (meth)acrylate copolymer was too small.

(Comparative Example 5)

A polyvinyl chloride resin (number-average polymerization degree: 2,500) (10 kg), 5 kg of DOP and 100 g of a (meth)acrylate copolymer 15 were charged and kneaded at 155°C for 3 hours. They were further subjected to a treatment at 160°C and 100 rpm using a biaxial extruder to prepare pellets. The resulting pellets were treated in the same manner as in Example 1 to prepare a tube.

In the tube prepared in this Comparative Example, its water drop width was "not good" probably due to the fact that the molecular weight of the (meth)acrylate copolymer was too low.

[Table 1]

| | Example 1 | Example 2 | Example 3 | Example 4 | Example 5 | Example 6 |
|---|---|---|---|---|---|---|
| polymerization degree of polyvinyl chloride resin | 1,000 | 2,000 | 2,500 | 2,500 | 2,500 | 2,500 |
| parts by weight of polyvinyl chloride resin | 100 | 100 | 100 | 100 | 100 | 100 |
| plasticizer | DOP | TOTM | DOP | DOP | DINCH | DOP |
| parts by weight of plasticizer | 10 | 30 | 50 | 110 | 110 | 50 |
| parts by weight of (meth)acrylate copolymer | 0.05 | 0.1 | 1 | 3 | 1 | 1 |
| molar ratio of alkyl (meth) acrylate | 65.8 | 56.1 | 75.7 | 71.5 | 66.1 | 67.2 |
| molar ratio of silicone (meth) acrylate | 0 | 0 | 0 | 0 | 0 | 1.7 |
| molar ratio of hydrophilic (meth) acrylate | 34.2 | 43.9 | 24.3 | 28.5 | 33.9 | 31.1 |
| number-average molecular weight of (meth)acrylate copolymer | 9,000 | 14,000 | 24,000 | 36,000 | 47,000 | 41,000 |
| carbon atom number of alkyl (meth)acrylate | 8 | 8 | 8 | 12 | 8 | 8 |
| silicone (meth)acrylate repeating unit(s) | - | - | - | - | - | 11 |
| hydrophilic (meth)acrylate repeating unit(s) | 3 | 3 | 3 | 3 | 3 | 3 |
| water drop width (cm) | 1.00 | 1.00 | 1.05 | 1.10 | 1.00 | 1.20 |
| glossiness | 42.5 | 40.1 | 38.3 | 36.2 | 37.6 | 39.5 |
| inner diameter (mm) | 6.4 | 6.4 | 6.4 | 6.4 | 6.4 | 6.4 |
| outer diameter (mm) | 11.0 | 11.0 | 11.0 | 11.0 | 11.0 | 11.0 |

[Table 2]

| | Example 7 | Example 8 | Example 9* | Example 10* | Example 11 | Example 12 |
|---|---|---|---|---|---|---|
| polymerization degree of polyvinyl chloride resin | 2,500 | 2,500 | 2,500 | 2,500 | 2,500 | 2,500 |

(continued)

| | Example 7 | Example 8 | Example 9* | Example 10* | Example 11 | Example 12 |
|---|---|---|---|---|---|---|
| parts by weight of polyvinyl chloride resin | 100 | 100 | 100 | 100 | 100 | 100 |
| plasticizer | DOP | DOP | DOP | DOP | DOP | DOP |
| parts by weight of plasticizer | 50 | 50 | 50 | 50 | 50 | 50 |
| parts by weight of (meth) acrylate copolymer | 1 | 1 | 1 | 1 | 1 | 1 |
| molar ratio of alkyl (meth) acrylate | 76.0 | 75.9 | 91.8 | 51.7 | 78.1 | 75.3 |
| molar ratio of silicone (meth) acrylate | 0 | 0 | 0 | 0 | 0 | 0 |
| molar ratio of hydrophilic (meth)acrylate | 24.0 | 24.1 | 8.2 | 48.3 | 21.9 | 24.7 |
| number-average molecular weight of (meth)acrylate copolymer | 29,000 | 25,000 | 25,000 | 25,000 | 34,000 | 23,000 |
| carbon atom number of alkyl (meth)acrylate | 18 | 4 | 8 | 8 | 8 | 8 |
| silicone (meth)acrylate repeating unit(s) | - | - | - | - | - | - |
| hydrophilic (meth)acrylate repeating unit(s) | 3 | 3 | 3 | 3 | 9 | 1 |
| water drop width (cm) | 0.90 | 1.30 | 0.90 | 1.45 | 150 | 0.95 |
| glossiness | 41.0 | 31.2 | 37.2 | 32.5 | 30.1 | 35.5 |
| inner diameter (mm) | 6.4 | 6.4 | 6.4 | 6.4 | 6.4 | 6.4 |
| outer diameter (mm) | 11.0 | 11.0 | 11.0 | 11.0 | 11.0 | 11.0 |
| *Reference Example | | | | | | |

[Table 3]

| | Comparative Example 1 | Comparative Example 2 | Comparative Example 3 | Comparative Example 4 | Comparative Example 5 |
|---|---|---|---|---|---|
| polymerization degree of polyvinyl chloride resin | 2,500 | 2,500 | 2,500 | 2,500 | 2,500 |
| parts by weight of polyvinyl chloride resin | 100 | 100 | 100 | 100 | 100 |
| plasticizer | DOP | DOP | DOP | DOP | DOP |
| parts by weight of plasticizer | 50 | 50 | 50 | 50 | 50 |
| parts by weight of (meth) acrylate copolymer | 1 | 1 | 6 | 0.001 | 1 |
| molar ratio of alkyl (meth) acrylate | 100 | 75.3 | 75.7 | 75.7 | 75.3 |

(continued)

| | Comparative Example 1 | Comparative Example 2 | Comparative Example 3 | Comparative Example 4 | Comparative Example 5 |
|---|---|---|---|---|---|
| molar ratio of silicone (meth)acrylate | 0 | 0 | 0 | 0 | 0 |
| molar ratio of hydrophilic (meth)acrylate | 0 | 24.7 | 24.3 | 24.3 | 24.7 |
| number-average molecular weight of (meth) acrylate copolymer | 25,000 | 60,000 | 24,000 | 24,000 | 6,000 |
| carbon atom number of alkyl (meth)acrylate | 8 | 8 | 8 | 8 | 8 |
| silicone (meth)acrylate repeating unit(s) | - | - | - | - | - |
| hydrophilic (meth)acrylate repeating unit(s) | - | 3 | 3 | 3 | 3 |
| water drop width (cm) | 0.85 | 1.05 | - | 0.85 | 0.85 |
| glossiness | 40.5 | 25.2 | - | 36.9 | 35.8 |
| inner diameter (mm) | 6.4 | 6.4 | - | 6.4 | 6.4 |
| outer diameter (mm) | 11.0 | 11.0 | - | 11.0 | 11.0 |

Industrial Applicability

Since the pellet-shaped composition for medical use according to the present invention contains an antithrombotic material in the composition, the after-treatment such as a surface treatment for the area contacting with blood after the molding is unnecessary whereby a big reduction in cost can be achieved. Moreover, when it is molded into a tube or the like, a molded product exhibiting neither coloration nor turbidity and having high transparency can be prepared whereby visibility in a tube can be ensured in the field of operation. Accordingly, it greatly contributes in the development of industry.

**Claims**

1. A pellet-shaped composition for medical use which comprises 1 to 120 part(s) by weight of a plasticizer and 0.01 to 5 part(s) by weight of a (meth)acrylate copolymer containing a hydrophobic (meth)acrylate unit and a hydrophilic (meth)acrylate unit to 100 parts by weight of a polyvinyl chloride resin, wherein number-average molecular weight of the (meth)acrylate copolymer is 7,000 to 50,000, and the hydrophobic (meth)acrylate unit and the hydrophilic (meth)acrylate unit are copolymerized in a molar ratio of 55 - 80 to 20 - 45.

2. The pellet-shaped composition for medical use according to claim 1, wherein the hydrophobic (meth)acrylate unit contains an alkyl (meth)acrylate unit represented by the following formula 1 and/or a silicone (meth)acrylate unit represented by the following formula 2:

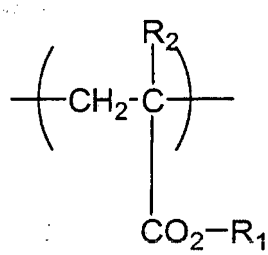

(1)

wherein, $R_1$ is an alkyl group or an aralkyl group having 6 to 14 carbon atoms and $R_2$ represents hydrogen atom or methyl group;

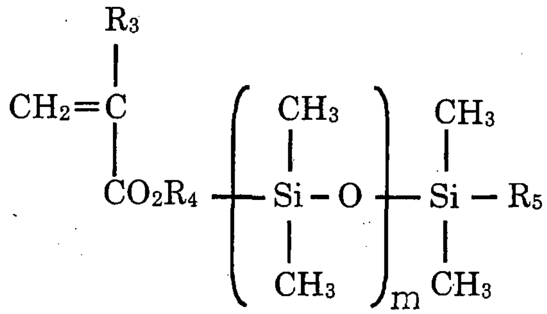

(2)

wherein, $R_3$ represents hydrogen atom or methyl group, $R_4$ represents an alkylene group having 1 to 6 carbon atom(s), $R_5$ represents an alkyl group having 1 to 6 carbon atom(s) and m is within a range of 1 to 100.

**3.** The pellet-shaped composition for medical use according to claim 1 or 2, wherein the hydrophilic (meth)acrylate unit contains a methoxy polyethylene glycol (meth)acrylate unit represented by the formula 3:

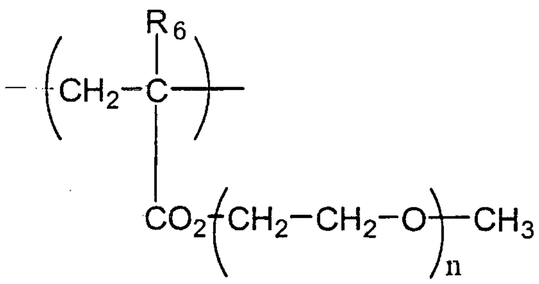

(3)

wherein, $R_6$ represents hydrogen atom or methyl group and m is an integer of 2 to 4.

**4.** The pellet-shaped composition for medical use according to any of claims 1-3, wherein number-average polymerization degree of the polyvinyl chloride resin is 700 to 3,000.

**5.** The pellet-shaped composition for medical use according to any of claims 1-4, wherein the plasticizer is one or more member(s) selected from di-2-ethylhexyl phthalate (DOP), tri-2-ethylhexyl trimellitate (TOTM) and diisononyl cyclohexane-1,2-dicarboxylate (DINCH).

**6.** A molded product prepared by melt molding the pellet-shaped composition for medical use mentioned in any of claims 1-5.

**7.** A tube for medical use prepared by melt molding the pellet-shaped composition for medical use mentioned in any of claims 1-5.

## Patentansprüche

**1.** Eine pelletförmige Zusammensetzung zur medizinischen Verwendung, welche 1 bis 120 Gewichtsteile eines Weichmachers und 0,01 bis 5 Gewichtsteile eines (Meth)acrylat-Copolymers, das eine hydrophobe (Meth)acrylat-Einheit und eine hydrophile (Meth)acrylat-Einheit enthält, bezogen auf 100 Gewichtsteile eines Polyvinylchloridharzes umfasst, wobei das Zahlenmittel des Molekulargewichts des (Meth)acrylat-Copolymers 7.000 bis 50.000 beträgt und die hydrophobe (Meth)acrylat-Einheit und die hydrophile (Meth)acrylat-Einheit in einem Molverhältnis von 55 - 80 zu 20 - 45 copolymerisiert sind.

**2.** Die pelletförmige Zusammensetzung zur medizinischen Verwendung nach Anspruch 1, wobei die hydrophobe (Meth)acrylat-Einheit eine Alkyl(meth)acrylat-Einheit, dargestellt durch die folgende Formel 1, und/oder eine Silicon(meth)acrylat-Einheit, dargestellt durch die folgende Formel 2, enthält:

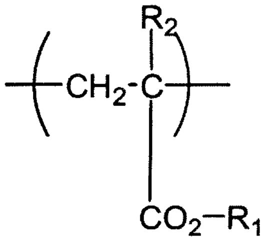

(1)

wobei $R_1$ eine Alkylgruppe oder eine Aralkylgruppe mit 6 bis 14 Kohlenstoffatomen ist und $R_2$ ein Wasserstoffatom oder eine Methylgruppe bedeutet;

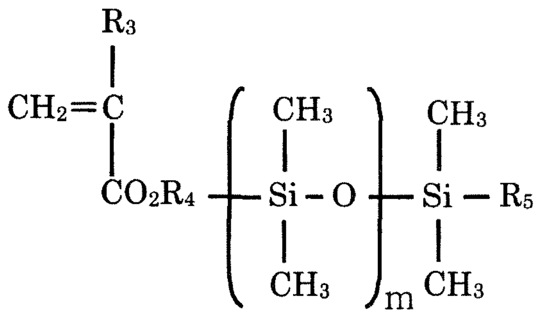

(2)

wobei $R_3$ ein Wasserstoffatom oder eine Methylgruppe bedeutet, $R_4$ eine Alkylengruppe mit 1 bis 6 Kohlenstoffatomen bedeutet, $R_5$ eine Alkylgruppe mit 1 bis 6 Kohlenstoffatomen bedeutet und m in einem Bereich von 1 bis 100 liegt.

**3.** Die pelletförmige Zusammensetzung zur medizinischen Verwendung nach Anspruch 1 oder 2, wobei die hydrophile (Meth)acrylat-Einheit eine Methoxypolyethylenglycol(meth)acrylat-Einheit, dargestellt durch die Formel 3, enthält:

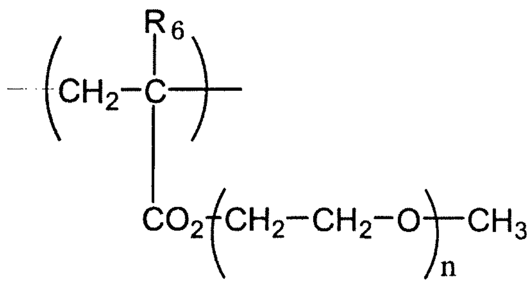

(3)

wobei $R_6$ ein Wasserstoffatom oder eine Methylgruppe bedeutet und m eine ganze Zahl von 2 bis 4 ist.

**4.** Die pelletförmige Zusammensetzung zur medizinischen Verwendung nach einem der Ansprüche 1-3, wobei das Zahlenmittel des Polymerisationsgrades des Polyvinylchloridharzes 700 bis 3.000 beträgt.

**5.** Die pelletförmige Zusammensetzung zur medizinischen Verwendung nach einem der Ansprüche 1-4, wobei der Weichmacher ein oder mehrere Mitglied(er), ausgewählt aus Di-2-ethylhexylphthalat (DOP), Tri-2-ethylhexyltrimellitat (TOTM) und Diisononylcyclohexan-1,2-dicarboxylat (DINCH), ist.

**6.** Ein Formprodukt, hergestellt durch Schmelzformen der pelletförmigen Zusammensetzung zur medizinischen Verwendung nach einem der Ansprüche 1-5.

**7.** Eine Röhre zur medizinischen Verwendung, hergestellt durch Schmelzformen der pelletförmigen Zusammensetzung zur medizinischen Verwendung nach einem der Ansprüche 1-5.

## Revendications

1. Composition sous forme de granulés à usage médical qui comprend de 1 à 120 parties en poids de plastifiant et de 0,01 à 5 parties en poids d'un copolymère de (méth)acrylate contenant un motif (méth)acrylate hydrophobe et un motif (méth)acrylate hydrophile pour 100 parties en poids de résine polychlorure de vinyle, dans laquelle le poids moléculaire moyen en nombre du copolymère de (méth)acrylate est de 7 000 à 50 000, et le motif (méth)acrylate hydrophobe et le motif (méth)acrylate hydrophile sont copolymérisés dans un rapport molaire de 55-80 à 20-45.

2. Composition sous forme de granulés à usage médical selon la revendication 1, dans laquelle le motif (méth)acrylate hydrophobe contient un motif (méth)acrylate d'alkyle représenté par la formule 1 suivante et/ou un motif (méth)acrylate de silicone représenté par la formule 2 suivante :

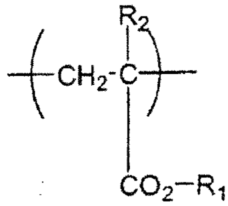

(1)

où, $R_1$ est un groupe alkyle ou un groupe aralkyle ayant de 6 à 14 atomes de carbone et $R_2$ représente un atome d'hydrogène ou un groupe méthyle ;

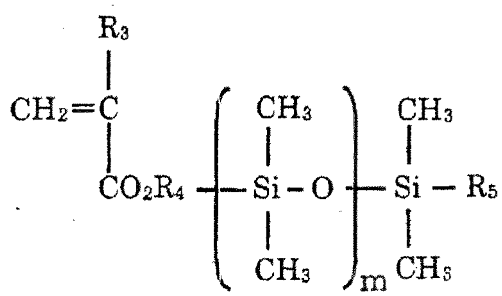

(2)

où, $R_3$ représente un atome d'hydrogène ou un groupe méthyle, $R_4$ représente un groupe alkylène ayant de 1 à 6 atomes de carbone, $R_5$ représente un groupe alkyle ayant de 1 à 6 atomes de carbone et m est dans une plage de 1 à 100.

3. Composition sous forme de granulés à usage médical selon la revendication 1 ou 2, dans laquelle le motif (méth)acrylate hydrophile contient un motif (méth)acrylate de méthoxypolyéthylène glycol représenté par la formule 3 :

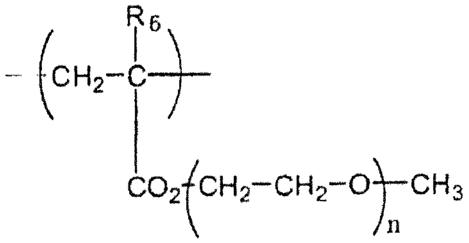

(3)

où, $R_6$ représente un atome d'hydrogène ou un groupe méthyle et m est un entier de 2 à 4.

4. Composition sous forme de granulés à usage médical selon l'une quelconque des revendications 1 à 3, dans laquelle le degré de polymérisation moyen en nombre de la résine polychlorure de vinyle est de 700 à 3 000.

5. Composition sous forme de granulés à usage médical selon l'une quelconque des revendications 1 à 4, dans laquelle le plastifiant est un ou plusieurs plastifiants choisis parmi le phtalate de di-2-éthylhexyle (DOP), le trimellitate de tri-2-éthylhexyle (TOTM) et le cyclohexane-1,2-dicarboxylate de diisononyle (DINCH).

6. Produit moulé préparé par moulage à l'état fondu de la composition sous forme de granulés à usage médical mentionnée dans l'une quelconque des revendications 1 à 5.

7. Tube à usage médical préparé par moulage à l'état fondu de la composition sous forme de granulés à usage médical mentionnée dans l'une quelconque des revendications 1 à 5.

[Fig. 1]

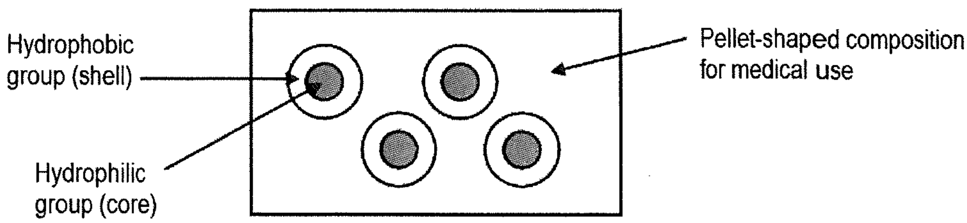


Micelle Formation of (Meth)acrylate Copolymer

[Fig. 2]

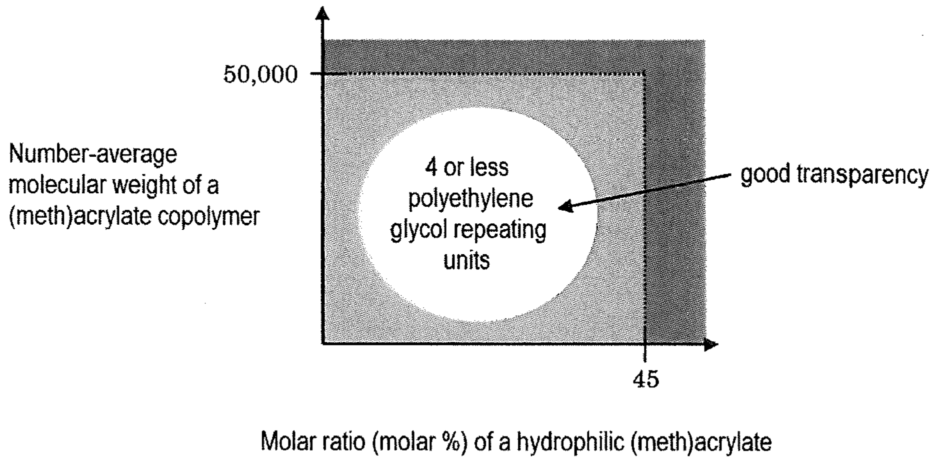


[Fig. 3]

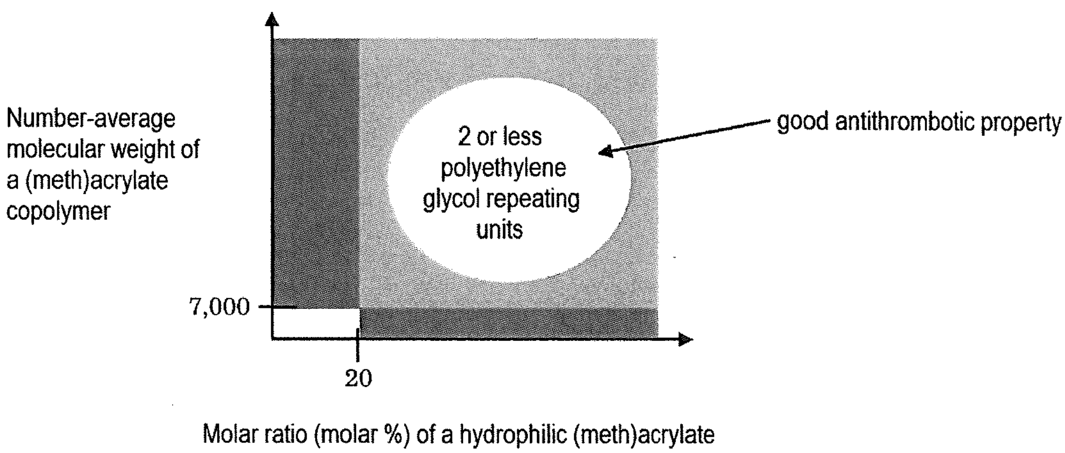

[Fig. 4]

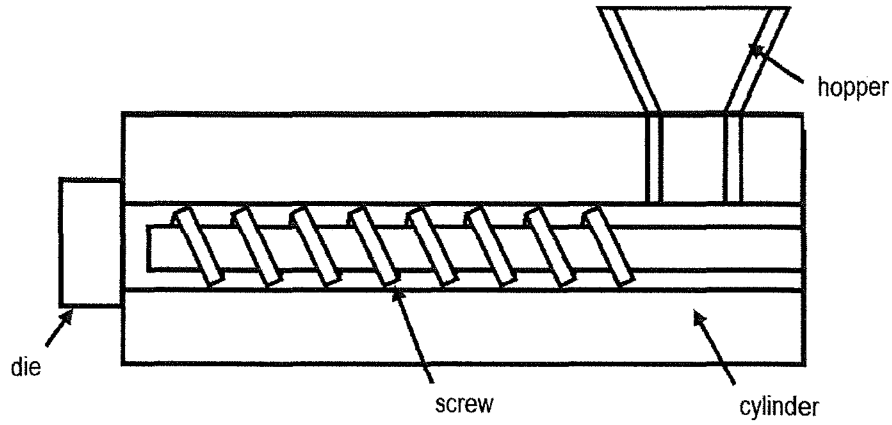
hopper
die
screw
cylinder

[Fig. 5]

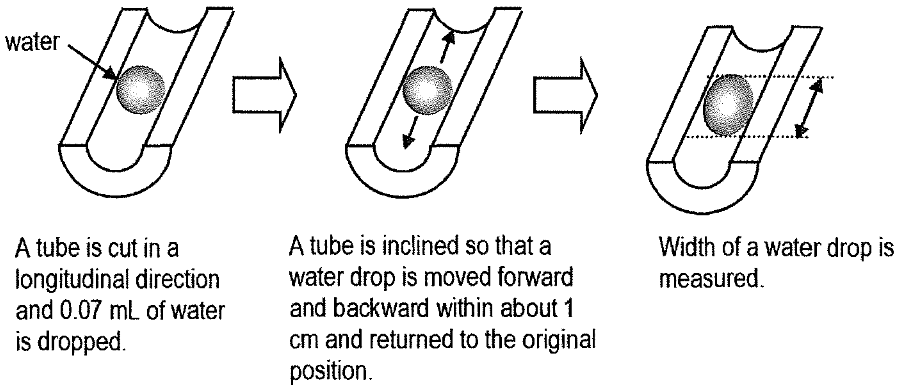
water
A tube is cut in a longitudinal direction and 0.07 mL of water is dropped.
A tube is inclined so that a water drop is moved forward and backward within about 1 cm and returned to the original position.
Width of a water drop is measured.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 3228409 B
- JP 4162028 B
- JP 4793700 B
- JP 2005089605 A
- JP 6116952 A


### Non-patent literature cited in the description


- POLYMER CHEMISTRY. OXFORD UNIVERSITY PRESS, 1999, 36